# EUROPÄISCHE PATENTSCHRIFT

(11) **EP 0 908 362 B1**
(45) Veröffentlichungstag und Bekanntmachung des Hinweises auf die Patenterteilung: **13.09.2006**
(21) Anmeldenummer: 98118257.9
(22) Anmeldetag: 25.09.1998
(51) Int. Cl.: B60S 1/38

(54) **Wischerblatt**
Wiper blade
Balai d'essuyage

(30) Priorität: 11.10.1997 DE 19745004
(43) Veröffentlichungstag der Anmeldung: 14.04.1999
(73) Patentinhaber: ROBERT BOSCH GMBH, 70442 Stuttgart (DE)
(72) Erfinder: Simons, Yvo, 3530 Houthalen (BE)

(56) Entgegenhaltungen:
- EP-A- 0 665 143
- DE-A- 3 936 597
- DE-A1- 4 322 465
- US-A- 3 317 945
- US-A- 5 568 670

## Beschreibung

Die Erfindung betrifft ein Wischerblatt, insbesondere für eine Wischanlage an Kraftfahrzeugen.

### Stand der Technik

Es sind Wischerblätter bekannt, die aus einem elastischen Material bestehen. Sie werden mittels eines Wischerarmes über eine zu wischende Scheibe eines Kraftfahrzeuges bewegt. Die Wischerblätter sind hierbei über einen Bügel mit dem Wischerarm gelenkig verbunden. Zur Anpassung an gewölbte Scheiben, die beispielsweise unterschiedliche Krümmungsradien aufweisen können, ist in die Wischerblätter eine Federschiene integriert, die über die Länge des Wischerblattes einen Druck in Richtung der zu wischenden Scheibe ausübt. Hierdurch wird eine gleichmäßige Anlage des Wischerblattes gewährleistet. Bekannt sind außenliegende Federschienen, die in entsprechende Nuten des Wischerblattes einbringbar sind. Da diese Federschienen den Witterungseinflüssen ausgesetzt sind, werden diese üblicherweise aus Edelstahl, beispielsweise Chrom-Nickel-Stahl, gefertigt.

Um Korrosionsprobleme der Federschienen zu vermeiden, ist es bekannt, sogenannte innenliegende Federschienen einzusetzen, die in einem Hohlraum des Wischerblattes angeordnet sind. Nachteilig sind die mit Edelstahlfederschienen verbundenen hohen Kosten.

Die US 5,568,670 A beschreibt ein Wischerblatt mit eingebetteter Federschiene, die durch die Einbettung geschützt wird. Weitere Korrosionsschutzmaßnahmen werden für die Federschiene nicht berücksichtigt.

Die Offenlegungsschrift DE 43 22 465 A1 beschreibt ein Stahlblech, welches eine Zink oder Zinklegierung aufweist, auf die zusätzlich als Temporärschutz eine Oxidschicht aufgedampft ist.

### Vorteile der Erfindung

Das erfindungsgemäße Wischerblatt mit den im Anspruch 1 genannten Merkmalen bietet demgegenüber den Vorteil, dass in einfacher Weise eine Korrosionsgefahr vermindert ist. Dadurch, dass die vom Wischerblatt eingeschlossene Federschiene aus einem Kohlenstoffstahl besteht, der mit einer aus einem höherwertigen Metall aufweisenden Beschichtung versehen ist, wobei die Beschichtung eine Zink-Aluminium-Legierung ist, die mit einem chromhaltigen Überzug versehen ist, ist ein möglichst umfassender Korrosionsschutz erlangt. Insbesondere bildet die Zink-Aluminium-Legierung mit dem Kohlenstoffstahl der Federschiene ein galvanisches Element Während die außenliegende chromhaltige Imprägnierschicht das Angreifen von zu Korrosionen führender Feuchtigkeit verhindert, wird an den Schnittstellen, das beispielsweise als strangförmiges Material hergestellten Federschienen, ein kathodischer Korrosionsschutz zwischen dem Kohlenstoffstahl, der Federschiene und der Zink-Aluminium-Legierung gewährleistet. Insgesamt kann somit gegenüber der Verwendung von Edelstahl für die Federschienen eine Kosteneinsparung bei Beibehaltung des Korrosionsschutzes gewährleistet werden.

In einer weiteren Ausgestaltung der Erfindung weist der Kohlenstoffstahl der Federschiene einen Kohlenstoffgehalt von 0,75 % auf.

### Zeichnung

Die Erfindung wird nachfolgend in einem Ausführungsbeispiel anhand der zugehörigen Zeichnung, die schematisch eine Schnittdarstellung durch ein Wischerblatt zeigt, näher erläutert.

### Beschreibung des Ausführungsbeispiels

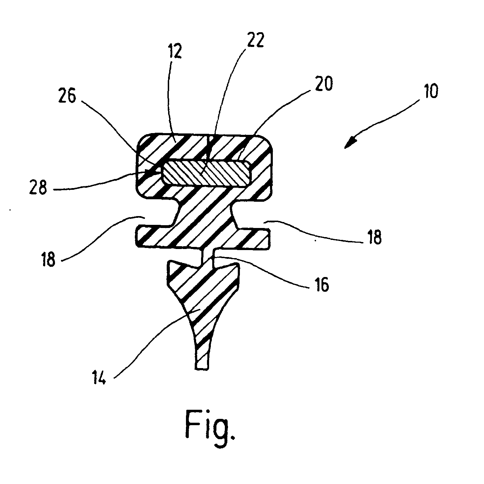

Die Figur zeigt eine Schnittdarstellung durch ein Wischerblatt 10. Das Wischerblatt 10 besteht aus einem Kopf 12, sowie einer Lippe 14, die mittels eines Umlenksteges 16 miteinander verbunden sind. Das Wischerblatt 10 besteht aus einem elastischen Material, beispielsweise Kautschukgummi. Der Kopf 12 besitzt in Längsrichtung des Wischerblattes 10 verlaufende Ausnehmungen 18, in die ein nicht dargestellter Bügel zur Befestigung des Wischerblattes 10 an einem Wischerarm eingreift. Innerhalb des Kopfes 12 ist ein sich axial zum Wischerblatt 10 erstreckender Hohlraum 20 ausgebildet, der formschlüssig eine Federschiene 22 aufnimmt. Die Federschiene 22 besteht beispielsweise aus einem Flachdraht aus Kohlenstoffstahl, beispielsweise mit einem Kohlenstoffgehalt von 0,75 %. Der Flachdraht ist mit einer Beschichtung 26 versehen, die ein höherwertiges korrosionsbeständiges Metall, beispielsweise Zink Zn aufweist. Die Beschichtung 26 ist beispielsweise eine Zink-Aluminium-Legierung. Die Beschichtung 26 ist mit einem Überzug 28 aus einem chromhaltigen Imprägniermittel versehen.

Durch den Aufbau und die Materialwahl der Federschiene 22 wird erreicht, dass einerseits die federelastische Wirkung, die zu einem gleichmäßigen Andruck des Wischerblattes 10 auch an gewölbte Scheiben eines Kraftfahrzeuges führt, aufrechterhalten wird, und trotz des Einsatzes relativ preiswerten Kohlenstoffstahls ein ausreichender Korrosionsschutz gewährleistet ist. Die sich über die gesamte Länge der Federschiene 22 ausgebildete Beschichtung 26 und der Überzug 28 gewährleisten einen Schutz der Federschiene vor in den Hohlraum 20 des Wischerblattes 10 eintretender Feuchtigkeit. An den stirnseitigen Schnittkanten der Federschiene 22 wird durch die Beschichtung 26 zwischen dem Kohlenstoffstahl der Federschiene 22 und dem Zinkanteil der Beschichtung 26 ein galvanisches Element ausgebildet, das zu einem kathodischen Korrosionsschutz führt. Hierbei wird in bekannter Weise ein beim Korrosionsvorgang fließender elektrischer Strom durch einen entgegengesetzten gleichgroßen elektrischen Strom kompensiert. Die Zinkanteile der Beschichtung 26 wirken hierbei als Opferanode, so daß eine Standzeit der Federschiene 22 deutlich über einer Standzeit des gesamten Wischerblattes 10 liegt.

Durch den zusätzlichen chromhaltigen Überzug 28 wird darüber hinaus ein Reibungskoeffizient zwischen der Federschiene 22 und dem Wischergummi 10 verringert, so dass hierdurch eine einfache Montierbarkeit, durch Einschieben der Federschiene 22 in den Hohlraum 20 gewährleistet ist, und andererseits während des bestimmungsgemäßen Einsatzes des Wischerblattes 10 eine Leichtgängigkeit während der auftretenden Relativverschiebung zwischen dem Kopf 12 des Wischerblattes 10 und der Federschiene 22 gewährleistet ist.

## Patentansprüche

1. Wischerblatt, insbesondere für eine Wischanlage an Kraftfahrzeugen, mit einer von dem Wischerblatt eingeschlossenen, sich im wesentlichen über die gesamte Länge des Wischerblattes erstreckenden Federschiene wobei die Federschiene (22) aus einem Kohlenstoffstahl besteht, **dadurch gekennzeichnet, daß** der Kohlenstoffstahl mit einer aus einem höherwertigen Metall aufweisenden Beschichtung (26) versehen ist, die Beschichtung (26) eine Zink-Aluminium-Legierung ist und die Beschichtung (26) mit einem chromhaltigen Überzug (28) versehen ist.

2. Wischerblatt nach Anspruch 1, **dadurch gekennzeichnet, dass** der Kohlenstoffstahl einen Kohlenstoffgehalt von 0,75 % aufweist.

## Claims

1. Wiper blade, in particular for a wiper system on motor vehicles, with a spring rail which is enclosed by the wiper blade and extends essentially over the entire length of the wiper blade, the spring rail (22) being composed of a carbon steel, **characterized in that** the carbon steel is provided with a coating (26) having a higher-quality metal, the coating (26) is a zinc-aluminium alloy, and the coating (26) is provided with a chrome-containing protective layer (28).

2. Wiper blade according to Claim 1, **characterized in that** the carbon steel has a carbon content of 0.75%.

## Revendications

1. Lame d'essuyage notamment pour une installation d'essuie-glace de véhicules automobiles, comportant un rail élastique inséré dans la lame d'essuyage et s'étendant pratiquement sur toute la longueur de la lame d'essuyage, le rail élastique (22) étant réalisé en un acier au carbone,
**caractérisée en ce que**
l'acier au carbone est muni d'un revêtement (26) en un métal plus noble,
le revêtement (26) est un alliage zinc-aluminium et le revêtement (26) est muni d'une couverture (28) contenant du chrome.

2. Lame d'essuie-glace selon la revendication 1,
**caractérisée en ce que**
l'acier au carbone a une teneur en carbone de 0,75 %.
